(19) Europäisches Patentamt
European Patent Office
Office européen des brevets

(11) EP 2 430 981 A1

(12) **EUROPEAN PATENT APPLICATION**
published in accordance with Art. 153(4) EPC

(43) Date of publication:
21.03.2012 Bulletin 2012/12

(51) Int Cl.:
*A61B 10/00* (2006.01)   *G01N 21/35* (2006.01)

(21) Application number: 10774970.7

(22) Date of filing: 13.05.2010

(86) International application number:
PCT/JP2010/058119

(87) International publication number:
WO 2010/131713 (18.11.2010 Gazette 2010/46)

(84) Designated Contracting States:
AL AT BE BG CH CY CZ DE DK EE ES FI FR GB GR HR HU IE IS IT LI LT LU LV MC MK MT NL NO PL PT RO SE SI SK SM TR

(30) Priority: 13.05.2009 JP 2009116794

(71) Applicants:
• Sumitomo Electric Industries, Ltd.
Chuo-ku
Osaka-shi
Osaka 541-0041 (JP)
• Kyoto University
Sakyo-ku
Kyoto-shi
Kyoto 606-8501 (JP)

(72) Inventors:
• OKADA Kazunori
Yokohama-shi
Kanagawa 244-8588 (JP)
• SUGANUMA Hiroshi
Yokohama-shi
Kanagawa 244-8588 (JP)
• ISHII Akira
Kyoto-shi
Kyoto 606-8501 (JP)
• MUNEMITSU Toshihiro
Kyoto-shi
Kyoto 606-8501 (JP)

(74) Representative: HOFFMANN EITLE
Patent- und Rechtsanwälte
Arabellastraße 4
81925 München (DE)

(54) **BLOOD VESSEL INNER WALL ANALYZING DEVICE AND BLOOD VESSEL INNER WALL ANALYZING METHOD**

(57) The present invention relates to a blood vessel inner wall analyzing apparatus provided with a structure for more accurately analyzing components of substances adhered to the inner walls of blood vessels. A blood vessel inner wall analyzing apparatus (1) is provided with illuminating means, detecting means and analyzing means. The illuminating means illuminates a light component in a measurement wavelength range of 1957 nm to 2713 nm onto a measured position from an end surface (30b) of a light illuminating fiber (30) inserted into a carotid artery (C). The detecting means receives a light component from a measured position through an end surface (40a) of a light receiving fiber (40) within the carotid artery (C). The light component to be detected is a light component in a detection wavelength range which extends 15 nm on both short and long sides of the selected center wavelength, with each one or more center wavelengths being selected from a wavelength group comprised of specific wavelengths respectively in the measurement wavelength range. The analyzing means analyzes for the presence or absence of substances differing from blood by using intensity information on the detected light component.

Fig.1

## Description

### Technical Field

The present invention relates to a blood vessel inner wall analyzing apparatus suitable for analyzing compositions of a measured position within a blood vessel, and to a blood vessel inner wall analyzing method.

### Background Art

Carotid artery plaque is known to be an important factor that induces cerebral infarction. Carotid artery plaque is an attached substance that is formed on the inner walls of the carotid arteries by blood cholesterol, neutral fats and the like. In this case, the attached substance separates from an inner wall of a carotid artery and obstructs a portion of an intracerebral blood vessel, it can cause a serious disorder such as cerebral infarction. Consequently, known methods used for early diagnosis of the status of the inner walls of blood vessels include diagnostic methods using echo ultrasound from outside the body, and diagnostic methods comprising inserting a catheter having a measuring probe attached thereto into a blood vessel.

There are several types of the above-mentioned plaque, including those having a lipid core comprised of cholesterol and atheromas formed from leukocytes and their remains. In the case an atheroma, which is one type of plaque, is ruptured due to some form of pressure or separates from a blood vessel inner wall, serious disorders such as cerebral infarction are known to be induced at a high probability. Therefore, it is desirable to identify the type of plaque in order to more accurately diagnose the status of blood vessel inner walls, and in response to this desire, plaque identification methods have been examined in the manner of those described in Patent Documents 1 and 2, for example. For example, Patent Document 1 discloses a method for inferring the compositions of plaque from the hardness of the plaque determined on the basis of changes in shape caused by pulsation by continuously observing the plaque with a measuring probe inserted into a blood vessel. In addition, Patent Document 2 discloses a method for inferring the composition of plaque by illuminated light components having two or more different types of properties onto a measured position, receiving the reflected/scattered light from the measured position, creating a phantom of each optical plaque, and comparing measurement results for the plaque obtained by echo ultrasound with the phantom.

### Citation List

#### Patent Document

Patent Document 1: Japanese Patent Application Laid-Open No. 2004-329550
Patent Document2: Japanese Patent Application Laid-Open No. 2007-185242

#### Non-Patent Document

Non-Patent Document 1: Chenan Xia, et al., "Mid-infrared supercontinuum generation to 4.5 $\mu$m in ZBLAN fluoride fibers by nanosecond diode pumping", Optics Letters, Vol. 31, No. 17, pp. 2553-2555, September 1, 2006.

### Disclosure of the Invention

### Problems that the Invention is to Solve

The present inventors have examined conventional plaque measurement methods as described above, and as a result, have discovered the following problems.

Namely, in the identification method described in the above-mentioned Patent Document 1, compositions of plaque are inferred based on changes in the shape of the plaque caused by pulsation that varies considerably depending on the individual subject. Consequently, the identification method described in Patent Document 1 cannot be said to be a quantitative measurement method, and accurate evaluation of plaque compositions with the method is not considered to be easy. In addition, in the above-mentioned Patent Document 2, there is no disclosure regarding the specific method by which the plaque phantom is formed, and it is therefore considered to be difficult to create a phantom corresponding to the type of plaque. Consequently, accurate identification of the type of substance attached to a blood vessel inner wall such as plaque is considered to be difficult with the identification method described in Patent Document 2.

The present invention has been developed to eliminate the problems described above. It is an object of the present invention to provide a blood vessel inner wall analyzing apparatus provided with a structure for analyzing components of substances attached to blood vessel inner walls more accurately, and a blood vessel inner wall analyzing method.

**Means for Solving the Problems**

In order to achieve the above-mentioned object, the blood vessel inner wall analyzing apparatus according to the present invention is used to analyze compositions of a measured position within a blood vessel, and more specifically, is provided with illuminating means, detecting means and analyzing means.

The illuminating means illuminates at least a light component in a measurement wavelength range of 1957 nm to 2713 nm onto the measured position within the blood vessel. In addition, the illuminating means includes a light emitting end inserted into the blood vessel, and a light source that supplies the light component to be illuminated onto the measured position through the light emitting end. The detecting means includes a light entering end to be provided within the blood vessel, and a detector that detects from the measured position the light component introduced through the light entering end, in order to detect the light component from the measured position. The analyzing means analyzes compositions of the measured position based on intensity information on the light component detected by the detecting means.

In particular, in the blood vessel inner wall analyzing apparatus according to the present invention, the detecting means detects the light component in a detection wavelength ranges which extends 15 nm on both short and long sides of a selected center wavelength, with each of one or more center wavelengths being selected from a wavelength group comprised of 1972, 2055, 2057, 2141, 2167, 2172, 2182, 2212, 22577 2259, 2270, 2274, 2282, 2289, 2294, 2309, 2311, 2324, 2334, 2345, 2348, 2360, 2371, 2375, 2385, 2388, 2402, 2416, 2443, 2468, 2469, 2488, 2504, 2510, 2544, 2565, 2607, 2628, 2630, 2656, 2668, 2678 and 2697 nm. The analyzing means analyzes for the presence or absence of substances differing from blood in the blood vessel by analyzing compositions of the measured position using intensity information on the light component in the detection wavelength range.

In accordance with the blood vessel inner wall analyzing apparatus having the configuration described above, although the light component in the measurement wavelength range of 1957 nm to 2713 nm is illuminated onto the measured position from the light emitting end of the illuminating apparatus inserted into the blood vessel, the light component from the measured position is detected through the light entering end of the detecting apparatus inserted into the blood vessel. Here, the light component from the measured position, in the detection wavelength range which extends 15 nm on both short and long sides of the selected center wavelength, with each of one or more center wavelengths being selected from the wavelength group described above, indicates characteristics that differ according to the compositions of the measured position. The analyzing means analyzes for the presence or absence of substances differing from blood using light components for each of the above-mentioned detection ranges by utilizing these optical characteristics that differ for each composition in this manner. As a result, the blood vessel inner wall analyzing apparatus is able to more accurately analyze for the presence or absence of substances such as plaque that differ from blood in blood vessels.

In addition, the blood vessel inner wall analyzing apparatus according to the present invention may also use a supercontinuum light source for the light source of the illuminating means. The use of a supercontinuum light source, which is able to output broadband light at high intensity to the blood vessel inner wall analyzing apparatus without generating heat, makes it possible to analyze compositions of blood vessel inner walls with higher precision.

Here, the blood vessel inner wall analyzing apparatus according to the present invention may also be provided with a spectrometer arranged in the light path from the light source of the illuminating means to the detector of the detecting means. This spectrometer separates at least the light component within the detection wavelength range of the output light from the light source. Furthermore, the spectrometer constitutes a part of the illuminating means in a configuration in which it is arranged in the light path between the light source and the light emitting end. In addition, the spectrometer constitutes a part of the detecting means in a configuration in which it is arranged in the light path between the measured position and the light detector.

In one aspect for achieving the above-mentioned object, for example, the detecting means detects the light component in the detection wavelength range which extends 15 nm on both short and long sides of the selected center wavelength, with each of one or more center wavelengths being selected from the wavelength group comprised of 1972, 2057, 2141, 2167, 2182, 2212, 2257, 2274, 2289, 2311, 2345, 2388, 2416, 2469, 2504, 2544 and 2630 nm. In this case, the analyzing means analyzes for the presence or absence of thrombi and hematomas in the blood vessel by analyzing compositions of the measured position.

In addition, the detecting means may also detect the light component in the detection wavelength range which extends 15 nm on both short and long sides of the selected center wavelength, with each of one or more center wavelengths being selected from the wavelength group comprised of 2055, 2172, 2259, 2270, 2282, 2294, 2309, 2324, 2334, 2348, 2360, 2371, 2375, 2385, 2402, 2443, 2468, 2488, 2150, 2565, 2607, 2628, 2656, 2668, 2678 and 2697 nm. In this case, the analyzing means analyzes for the presence or absence of plaque in the blood vessel by analyzing compositions of the measured position.

As described above, substances other than blood present within blood vessels consisting of thrombi, hematomas and plaque indicate characteristics for which light components in the detection wavelength range which extends 15 nm on both short and long sides of the selected center wavelength, with each of one or more center wavelengths being selected from the above-mentioned wavelength groups, respectively differ according to the compositions of a measured position. The analyzing means utilizes these optical characteristics differing for each composition in this manner, and the analyzing means is able to more accurately analyze for the presence or absence of thrombi, hematomas and plaque by using these light components within a detection wavelength range.

In another mode to achieve the object of the present invention, for example, the detecting means detects, for three or more center wavelengths at least one of which is selected from the above-mentioned wavelength group, the light components belonging to the detection wavelength ranges which extend 15 nm on both short and long sides of the center wavelength. On the other hand, the analyzing means calculates a spectral shape parameter to be used for identifying composition of the measured position, and analyzes the composition based on the results of the calculation, wherein the spectral shape parameter is either first derivative, normalized first derivative, second derivative or normalized second derivative of intensity information in each of the detection wavelength ranges and is calculated at the center wavelength.

More specifically, in the case the light component belonging to the detection wavelength range which extends 15 nm on both short and long sides of the wavelength of 1972 nm is detected by the detecting means, the analyzing means is able to analyze for the absence or presence of thrombi in the blood vessel by analyzing composition of a measured position. Furthermore, in the case, for one or more center wavelengths selected from the wavelength group comprised of 2309 nm and 2628 nm, the light components belonging to the detection wavelength ranges which extend 15 nm on both short and long sides of the center wavelengths are detected by the detecting means, the analyzing means is able to analyze for the presence or absence of an atheromas in a blood vessel by analyzing composition of a measured position. Moreover, in the case, for one or more center wavelengths selected from the wavelength group comprised of 2371. nm and 2402 nm, the light components belonging to the detection wavelength ranges which extend 15 nm on both short and long sides of the center wavelengths are detected by the detecting means, the analyzing means is able to analyze for the presence or absence of a lipid core in a blood vessel by analyzing composition of a measured position.

The blood vessel inner wall analyzing apparatus according to the present invention may also be further provided with a display means that displays an analysis result obtained from the analyzing means in real time. In this case, analyses can be carried out while confirming analysis results with the display means, for example, thereby making it possible to enhance ease of use of the blood vessel inner wall analyzing apparatus by a user.

In addition, the blood vessel inner wall analyzing apparatus according to the present invention analyzes compositions of the measured position in a blood vessel by using the blood vessel inner wall analyzing apparatus having the previously described configuration. More specifically, in the blood vessel inner wall analyzing apparatus, prior to measurement, together with the light emitting end being inserted into the blood vessel so as to be positioned near the measured position, the light entering end is installed at a predetermined position in the blood vessel reached by the light component from the measured position. Following completion of installation of the light emitting end and the light entering end, the light component at least in the measurement wavelength range of 1957 nm to 2713 nm is illuminated onto the measured position through the light emitting end, and the light component that has entered through the light entering end is detected from the measured position. When a desired light component is detected, analysis data is generated for identifying a composition of the measured position from intensity information on the detected light component.

More specifically, in the blood vessel inner wall analyzing method according to the present invention, the light components belonging to the detection wavelength ranges which extend 15 nm on both short and long sides of the center wavelengths, for three or more center wavelengths at least one of which is selected from the above-mentioned wavelength group, is detected, and a spectral shape parameter to be used for identifying composition of the measured position is calculated, wherein the spectral shape parameter is either first derivative, normalized first derivative, second derivative or normalized second derivative of intensity information in each of the detection wavelength ranges and is calculated at the central wavelength.

In addition, in the blood vessel inner wall analyzing method according to the present invention, color-coded, two-dimensional tomographic images of blood vessels are displayed in real time for each composition of a measured position.

**Effects of the Invention**

In accordance with the blood vessel inner wall analyzing apparatus and blood vessel inner wall analyzing method according to the present invention, components or substances adhered to the inner wall of a blood vessel can be more accurately analyzed.

**Brief Description of the Drawings**

Fig. 1 is a drawing showing the configuration of an embodiment of a blood vessel inner wall analyzing apparatus according to the present invention;

Fig. 2 is a drawing for explaining plaque on the inner wall of a blood vessel;

Fig. 3 shows the absorption spectra of blood and thrombus;

Fig. 4 shows the second derivatives of the absorption spectra of blood and thrombus (Fig. 3);

Fig. 5 shows the absorption spectra of a blood vessel wall (inner membrane) and plaque (atheroma, lipid core, fibrous layer);

Fig. 6 shows the second order derivatives of the absorption spectra of a blood vessel wall (inner membrane) and plaque (atheroma, lipid core, fibrous layer) (Fig. 5); and

Fig. 7 is a drawing showing display example of analysis results obtained by a blood vessel inner wall analyzing apparatus.

**Best Modes for Carrying Out the Invention**

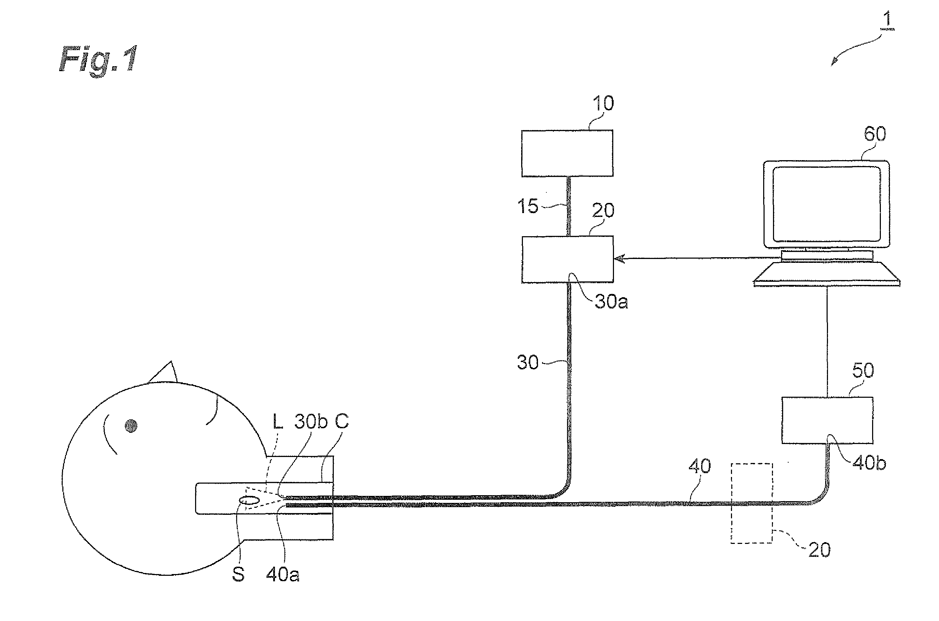

In the following, embodiments of the blood vessel inner wall analyzing apparatus and blood vessel inner wall analyzing method according to the present invention will be explained in detail with reference to Figs. 1 to 7. In the description of the drawings, identical or corresponding components are designated by the same reference numerals, and overlapping description is omitted.

(Configuration of Blood Vessel Inner Wall Analyzing Apparatus)

Fig. 1 is a drawing showing the configuration of an embodiment of the blood vessel inner wall analyzing apparatus according to the present invention. As shown in Fig. 1, a blood vessel inner wall analyzing apparatus 1 comprises a light source 10, a spectrometer 20, a light illuminating fiber 30, a light receiving fiber 40, a detection unit 50 and an analysis unit 60. In addition, the light source 10 and the spectrometer 20 are connected by an optical fiber 15. The detection unit 50 and the analysis unit 60 are electrically connected, while the analysis unit 60 and the spectrometer 20 are similarly electrically connected. In the blood vessel inner wall analyzing apparatus 1, the light source 10, the spectrometer 20 and the light illuminating fiber 30 constitute illuminating means that illuminates measurement light onto a measured position. The light receiving fiber 40 and the detection unit 50 constitute detecting means that detects a light component from the measured position. In addition, the analysis unit 60 functions as analyzing means for analyzing compositions of the measured position based on the intensity of light detected by the detecting means, and display means for displaying analysis results from the analyzing means.

The light source 10 is a light source that outputs near infrared light containing a wavelength range of 1957 nm to 2713 nm, and for example, a laser diode (LD) light source or a supercontinuum (SC) light source as described in the above-mentioned Non-Patent Document 1 is used. Among these, an SC light source is particularly preferable since it has the ability to illuminate a broad band of wavelengths at high output while also demonstrating little generation of heat from the light source per se. Light emitted from the light source 10 enters one of the end surfaces of the optical fiber 15, propagates through the optical fiber 15, and is inputted to the spectrometer 20.

The spectrometer 20 is inputted with light outputted from the light source 10 that has propagated through the optical fiber 15, and outputs to an optical fiber 25 only a light component within a detection wavelength range centering on a specific wavelength (such as a wavelength range extending 15 nm on either side of a specific central wavelength) based on instructions from the analysis unit 60. A diffraction grating or variable wavelength filter and the like is used for the spectrometer 20. In addition, in the case a diffraction grating is used for the spectrometer 20, the spectrometer 20 extracts a light component within a detection wavelength range centering on a specific wavelength and outputs the light component to the light illuminating fiber 30 by altering the inclination of the diffraction grating with respect to light entering from the light source 10 based on instructions from the analysis unit 60. Furthermore, the spectrometer 20 may also be arranged between the light receiving fiber 40 and the detection unit 50 so as to split light from a measured position (portion indicated with a broken line in Fig. 1).

The light illuminating fiber 30 is comprised of an optical fiber and the like, and is inserted into a carotid artery C during analysis of a blood vessel inner wall. A light component outputted from the spectrometer 20 is inputted as measurement light L from one end surface 30a of the light illuminating fiber 30, and is illuminated onto a measured position (such as an inner wall of the carotid artery C where plaque S is adhered) from the other end surface 30b inserted into the carotid artery C.

The light receiving fiber 40 is comprised of an optical fiber and the like, and allows propagation of a light component that has entered one end surface 40a among those light components that have been reflected/scattered as a result of illuminating the measurement light L onto the measured position. Subsequently, the light component is outputted from the other end surface 40b of the light receiving fiber 40 to the detection unit 50. A fiber bundle (imaging fiber), obtained by bundling a plurality of individual optical fibers, is preferably used as an optical fiber used for the light receiving fiber 40. In the case of using a fiber bundle for the light receiving fiber 40, the characteristics of the light component from the measured position can be measured two-dimensionally since the light component propagates through each individual optical fiber that constitutes the light receiving fiber 40.

The detection unit 50 detects a light component that has been input to the one end surface 40a of the light receiving fiber 40, propagated through the light receiving fiber 40, and is outputted from the other end surface 40b of the light receiving fiber. The detection unit 50 can use a light receiving element such as a photodiode, and converts a light component from a measured position to electrical current and then sends this converted electrical current to the analysis unit 60. Furthermore, the detection unit 50 can be provided at a location equivalent to the one end surface 40a of the light receiving fiber 40, and can employ a configuration in which a light component from a measured position is detected directly in the detection unit 50 without going through the light receiving fiber 40.

The analysis unit 60 analyzes a composition of a measured position based on intensity data of a light component detected by the detection unit 50, and displays the result on a monitor and the like. More specifically, the analysis unit 60 is comprised of a personal computer (PC) or workstation (WS) and the like, and is at least provided with display means such as a monitor, arithmetic processing means, input/output means and recording means. As a specific example of analysis carried out by the analysis unit 60, the analysis unit 60 calculates the intensity of light detected by the detection unit 50 for each wavelength based on the magnitude of electric current from a light receiving element of the detection unit 50. The analysis unit 60 then determines the intensity spectrum of the light component in the detection wavelength range from the calculation result for each wavelength, carries out second order differentiation on the intensity spectrum, and analyzes a composition of the measured position such as plaque based on the size of the peak originating in the composition of the measured position such as plaque. Furthermore, in addition to a second derivative of the intensity spectrum of each detection wavelength range as described above, the spectral shape parameter is also obtained according to, for example, a first derivative, normalized first derivative or normalized second derivative. The result is then notified to a user such as a subject by the PC or WS displaying the analysis result on a monitor and the like. In addition, a PC or WS having the function of the analysis unit 60 may also be provided with a function for controlling the entire blood vessel inner wall analyzing apparatus 1. Namely, the PC or WS has a function for selecting a wavelength of a light component to be split by the spectrometer 20 (central wavelength of the detection wavelength range) and issuing an instruction to the spectrometer 20.

Here, the second derivative of the intensity spectra in each detection wavelength range is one of the spectral shape parameters for identifying a composition of the measured position, and can be calculated using the following method. Namely, intensity data (spectral data) detected in each detection wavelength range respectively centering on the three detection wavelengths of x-b, x and x+a is defined as f(x-b), f(x) and f(x+a), and a value g(x) calculated in the following equation (1) is used as an approximate value of the second derivative at wavelength x.

$$g(x) = \frac{bf(x+a) + af(x-b) - (a+b)f(x)}{ab(a+b)/2} \quad \cdots (1)$$

At this time, the error from the true second derivative f''(x) becomes that determined with the following equation (2):

$$g(x) - f''(x) = \frac{1}{3}f'''(x)(a-b) + \frac{1}{12}f^{(4)}(x)(a^2 - ab + b^2) \quad \cdots (2)$$

wherein, parameters starting with the third order and above relating to a and b can be ignored.

Expected values for the third derivative f'''(x) and the fourth derivative f'(4)(x) on the right side of the above equation become zero since their signs and absolute values are unknown in ordinary measurements. Thus, the expected value of equation (2) becomes zero, and g(x) becomes an estimated amount having the least bias with respect to the second derivative. In addition, in the case it is preferable to calculate the first derivative of each intensity spectrum detected in the detection wavelength range as a spectral shape parameter, the value h(x) calculated using the following equation (3) is similarly used as an approximate value of the first derivative.

$$h(x) = \frac{b^2 f(x+a) - a^2 f(x-b) - (b^2 - a^2) f(x)}{ab(a+b)} \qquad \cdots (3)$$

At this time, the error from the true first derivative f'(x) becomes that determined with the following equation (4):

$$h(x) - f'(x) = \frac{1}{6} f'''(x) ab \qquad \cdots (4)$$

wherein, parameters starting with the third order and above relating to a and b can be ignored.

Similar to the discussion relating to the second derivative, h(x) becomes an estimated amount having the least bias with respect to the first derivative.

Furthermore, in the example described above, spectral shape parameters are calculated using intensity data in each detection wavelength range using three wavelengths as central wavelengths. In addition, although a second derivative and first derivative of the intensity spectra are indicated as spectral shape parameters, other spectral shape parameters may also be defined by revising the calculation formulas. For example, the first derivative and second derivative may be normalized by the average values of intensity at the three wavelengths. In addition, measurement accuracy can be improved by increasing the number of wavelengths used. In this case, a known numerical differentiation method such as the Savitzky-Golay method is used preferably. On the other hand, a method in which approximate values of the second derivative and first derivative are calculated using only three wavelengths as in the example described above, is suitable for displaying calculation results in real time since there is little time required for measurement and arithmetic processing. In particular, when displaying calculation results as real-time images of pseudo-colors, by assigning spectral shape parameters calculated from three wavelengths in the manner of the above-mentioned g(x) or h(x) to a single component of pseudo-colors consisting of the three primary colors, images of information reflecting the composition of a measurement target can be displayed in real time. As a result, the time required for diagnosis can be shortened, and the physical burden on a patient can be reduced. This is particularly important in the case of comparatively invasive diagnostic procedures performed in the body as is the case with observation of blood vessel walls.

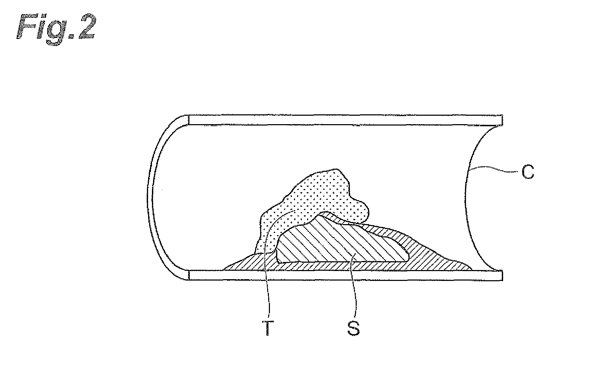

Measurement targets of the blood vessel inner wall analyzing apparatus 1 include plaque, thrombi and hematomas adhered to the inner wall of the carotid artery C. Four typical examples of plaque adhered to the inner wall of the carotid artery C include lipid cores comprised of cholesterol, atheromas formed from leukocytes and their remains, calculi resulting from the calcification of cholesterol-like substances, and fibrous layers covering the surface of plaque comprised of the three components listed above and having collagen as the main component thereof. In addition, thrombi and hematomas comprised of blood clots within blood vessels also have the potential to be involved in brain diseases. Fig. 2 is a drawing schematically showing an example of the case of plaque S and a thrombus T adhered to the inner wall of the carotid artery C. In the case the plaque S and thrombus T have adhered to the inner wall of the carotid artery C, since the blood vessel diameter in the area of the carotid artery C where blood is able to flow through becomes narrow as shown in Fig. 2, this is known to cause stenosis and obstruction of the carotid artery C as well as cerebral infarction or cerebral ischemia and the like. Among the typical types of plaque described above, atheromas in particular are known to have the highest potential to cause cerebral infarction. On the other hand, since calculi and lipid cores have a low frequency of separating from the inner wall of the carotid artery C, plaque comprised of these components is known to have a low risk of obstructing intracerebral blood vessels and the like. Consequently, in order to more accurately determine the risk of occurrence of diseases such as cerebral infarction, it is thought to be necessary to identify the compositions of plaque adhered to the inner wall of the carotid artery C.

Whether or not plaque is adhered to the inner wall of the carotid artery C can be determined by measuring using diagnostic methods such as echo ultrasound diagnosis or intravascular ultrasound (IVUS) from outside the neck. However, in the case of echo ultrasound diagnosis or intravascular ultrasound, it is difficult to obtain information other than that relating to the shape of the plaque within the blood vessel, namely to identify plaque compositions. In addition, although thrombi and hematomas adhered to the inner wall of the carotid artery C and comprised of blood clots within blood vessels are also measurement targets for the blood vessel inner wall analyzing apparatus 1 since they also have the potential to be involved in brain diseases, their detection is difficult from outside the neck when using echo ultrasound or intravascular ultrasound.

In contrast, use of the blood vessel inner wall analyzing apparatus 1 according to the present embodiment makes it possible to detect plaque, thrombi and hematomas formed in the carotid artery C and identify their components, and the following provides an explanation of this using the measurement examples indicated below.

(Examples of Measurement Using Blood Vessel Inner Wall Analyzing Apparatus 1)

(Measurement Example 1)

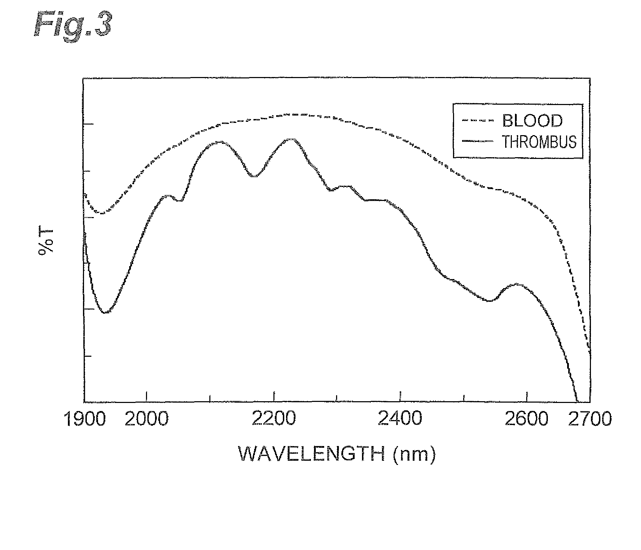

Infrared light over a measurement wavelength range of 1900 nm to 2700 nm was respectively illuminated as measurement light L onto blood and thrombi (thickness: 100 $\mu$m warmed to 38°C followed by measurement of their transmission spectra (%T). The results are shown in Fig. 3.

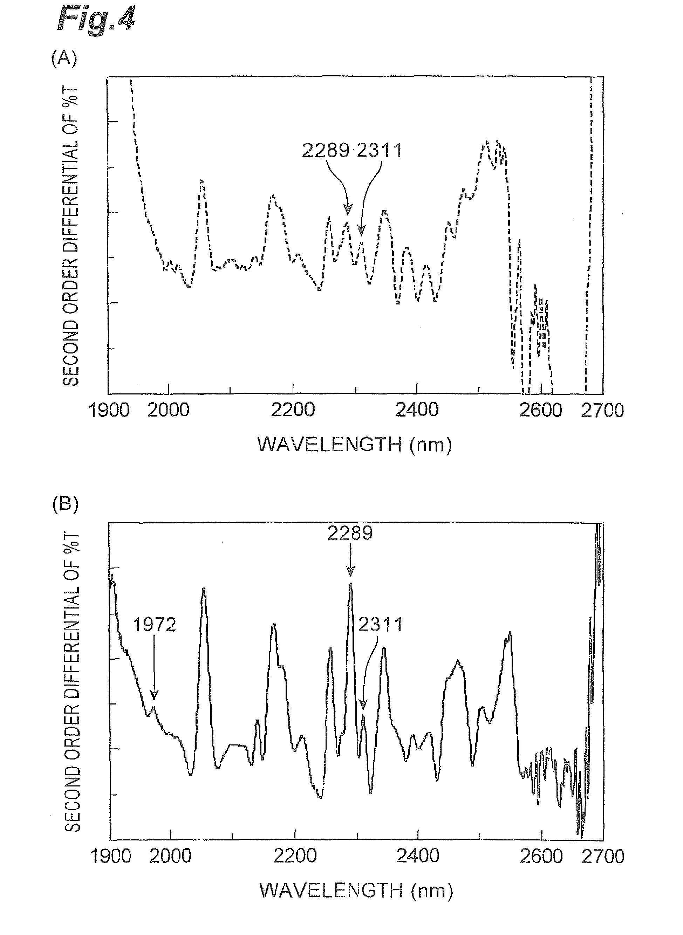

The results of second order differentiation of the blood and thrombi transmission spectra (%T) obtained from these measurements according to wavelength are shown in the areas (A) and (B) of Fig 4. In Fig. 4, the area (A) indicates the results of second order differentiation of the blood transmission spectrum according to wavelength, while the area (B) indicates the results of second order differentiation of the thrombi transmission spectrum according to wavelength. In this manner, characteristic peaks originating in each component were confirmed to be present between the blood and thrombi from the transmission spectra and the second derivatives thereof according to wavelength. More specifically, characteristic peaks for blood only, thrombi only or peaks common to both blood and thrombi were confirmed to be indicated at wavelengths of 1972, 2057, 2141, 2167, 2182, 2212, 2257, 2274, 2289, 2311, 2345, 2388, 2416, 2469, 2504, 2544 and 2630 nm. Thus, blood and thrombi were confirmed to be able to be distinguished based on the wavelength, height, height ratio or peak half-width and the like of the intensity spectra within each detection wavelength range which extends 15 nm on both short and long sides of each of these wavelengths as a center wavelength. In particular, both blood and thrombi can be distinguished using the presence or absence of a peak at the wavelength of 1972 nm, which is characteristic of thrombi, or using the height ratio of peaks at the wavelengths of 2289 nm and 2311 nm.

(Measurement Example 2)

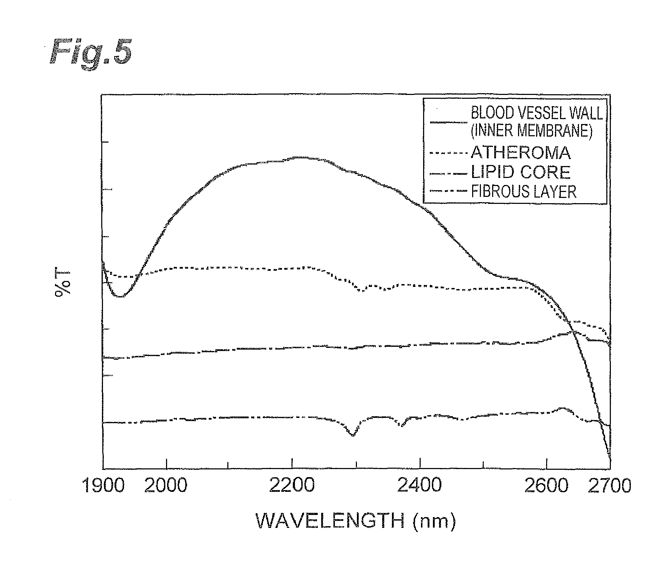

Infrared light over a measurement wavelength range of 1900 nm to 2700 nm was respectively illuminated as measurement light onto three types of plaque compositions consisting of atheroma, lipid core and fibrous layer (thickness: 100 $\mu$m) and a blood vessel wall (inner membrane) (thickness: 20 $\mu$m) each warmed to 38°C followed by measurement of their transmission spectra (%T). The results are shown in Fig. 5.

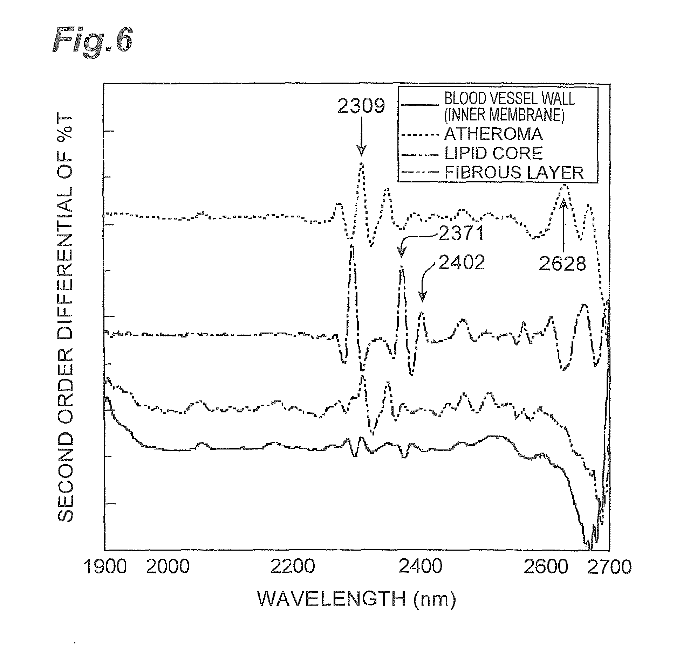

The results of second order differentiation of the respective transmission spectra (%T) of the plaque (three types) and blood vessel wall (inner membrane) obtained from these measurements according to wavelength are shown in Fig. 6. In this manner, characteristic peaks that mutually differed between each of the plaque components and the blood vessel wall (inner membrane) were confirmed to be present based on the transmission spectra and the second derivatives thereof according to wavelength. More specifically, characteristic peaks were confirmed to be indicated at wavelengths of 2055, 2172, 2259, 2270, 2282, 2294, 2309, 2324, 2334, 2348, 2360, 2371, 2375, 2385, 2402, 2443, 2468, 2488, 2510, 2565, 2607, 2628, 2656, 2668, 2678 and 2697 nm. Thus, distinction was confirmed to be able to be made between blood vessel wall (inner membrane) plaque as well as between the compositions of the plaque based on the wavelength, height, height ratio or peak half-width and the like of the spectra within each detection wavelength range which extends 15 nm on both short and long sides of each of these wavelengths as a center wavelength. In particular, atheroma and lipid core are thought to be able to be distinguished by using the presence or absence of peaks at wavelengths of 2309 nm and 2628 nm, which are characteristic of atheromas, and using the presence or absence of peaks at wavelengths of 2371 nm and 2402 nm, which are characteristic of lipid cores.

(Measurement Example 3)

A sample was produced by mixing blood containing an anticoagulant with thrombi. This sample was illuminated with measurement light outputted from an SC light source used for the light source 10 of the blood vessel inner wall analyzing apparatus 1 of the present embodiment. Scattered/reflected light from the sample was directed into a near infrared camera connected to a 15X Cassegrain magnifying glass (equivalent to a spectrometer and detector) to determine light intensity over the measurement wavelength range of 1000 nm to 2500 nm by hyperspectral measurement. This hyperspectral measurement refers to a measurement method whereby the spectrum is measured for each pixel. Among the measurement results obtained in this manner, when the presence or absence of a peak was confirmed at the wavelength of 1972 nm, the presence or absence of the peak was confirmed to differ for each measurement region (pixel). Since the peak at the wavelength of 1972 nm is the characteristic peak of thrombi, this measurement was confirmed to enable distinction of blood and thrombi.

(Measurement Example 4)

Hyperspectral measurement was carried out using plaque fragments for the sample and using the same measurement system as Measurement Example 3. Among the resulting measurement results, when the presence or absence of peaks was confirmed at the wavelengths of 2309, 2628, 2371 and 2402 nm, the presence or absence of the peaks was confirmed to differ for each measurement region (pixel). Since the peaks at the wavelengths of 2309 nm and 2628 nm and the peaks at the wavelengths of 2371 nm and 2402 nm are respectively peaks characteristic of atheromas and lipid cores that are typical components of plaque, this measurement was confirmed to enable identification of plaque compositions.

On the basis of the measurements described above, in the case of illuminating measurement light in the near infrared range onto a measured position, and measuring the spectrum of reflected/scattered light, blood and thrombi as well as blood vessel wall (inner membrane) and plaque (atheroma, lipid core, fibrous layer) were respectively confirmed to be able to be distinguished. Thus, compositions of substances adhered to the inner wall of the carotid artery C were confirmed to be able to be evaluated by inserting the light illuminating fiber 30 and the light receiving fiber 40 of the blood vessel inner wall analyzing apparatus 1 according to the present embodiment into the carotid artery C, illuminating the measurement light L onto a measured position from the end surface 30b of the light illuminating fiber 30 inserted into the carotid artery C, and detecting the light from the measured position that enters from the end surface 40a of the light receiving fiber 40 within the carotid artery C with the detection unit 50.

(Usage Examples of Blood Vessel Inner Wall Analyzing Apparatus)

As has been previously described, according to the blood vessel inner wall analyzing apparatus 1 according to the present invention, together with illuminating measurement light in the wavelength range of 1957 nm to 2713 nm onto the measured position from the end surface 30b of the light receiving fiber 30 inserted into the carotid artery C, light from the measured position enters the end surface 40a of the light receiving fiber 40 inserted into the carotid artery C and is detected. Here, the light components in the detection wavelength ranges each of which extends 15 nm on both short and long sides of the center wavelength, with each of one or more center wavelengths being selected from the wavelength group comprised of 1972, 2055, 2057, 2141 , 2167, 2172, 2182, 2212, 2257, 2259, 2270, 2274, 2282, 2289, 2294, 2309, 2311, 2324, 2334, 2345, 2348, 2360, 2371, 2375, 2385, 2388, 2402, 2416, 2443, 2468, 2469, 2488, 2504, 2510, 2544, 2565, 2607, 2628, 2630, 2656, 2668, 2678 and 2697 nm, demonstrates characteristics that differ according to compositions of the measured position. On the basis thereof, by analyzing for the presence or absence of substances differing from blood by using the light within these wavelength ranges, the presence or absence of substances differing from blood present in a blood vessel such as plaque can be more accurately analyzed.

The light components of the wavelength group comprised of 1972, 2057, 2141, 2167, 2182, 2212, 2257, 22744 2289, 2311, 2345, 2388, 2416, 2469, 2504, 2544 and 2630 nm are particularly suited for distinguishing between thrombi and blood. In addition, the light components of the wavelength group comprised of 2055, 2172, 2259, 2270, 2282, 2294, 2309, 2324, 2334, 2348, 2360, 2371, 2375, 2385, 2402, 2443, 2468, 2488, 2510, 2565, 2607, 2628, 2656, 2668, 2678 and 2697 nm are suited for analyzing compositions of plaque.

More specifically, the light component belonging to the detection wavelength range which extends 15 nm on both short and long sides of the wavelength of 1972 nm is preferable for analyzing for the presence or absence of thrombi in blood vessels. In addition, for one or more center wavelengths selected from the wavelength group comprised of 2309 nm and 2628 nm, the light components belonging to the detection wavelength ranges which extend 15 nm on both short and long sides of the center wavelengths, are preferable for analyzing for the presence or absence of atheromas in blood vessels. Moreover, for one or more center wavelengths selected from the wavelength group comprised of 2371 nm and 2402 nm, the light components belonging to the detection wavelength ranges which extend 15 nm on both short and long sides of the center wavelengths are preferable for analyzing for the presence or absence of lipid cores in blood vessels.

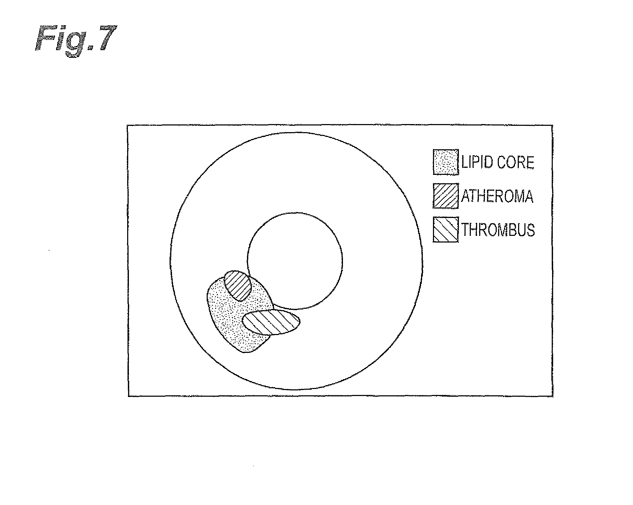

Analysis results from the blood vessel inner wall analyzing apparatus 1 are notified to a user such as a subject by displaying on a monitor provided in the analysis unit 60. In the case of using a fiber bundle (imaging fiber) for the light receiving fiber 40 through which propagates reflected light components or scattered light components from the measured position as in the present embodiment, a two-dimensional image can be obtained for each characteristic spectrum of plaque and the like. In addition, an affected area can be displayed by color coding, for example, by overlaying the resulting two-dimensional image on the analysis unit 60. As a result, the user can be notified in a more readily understandable manner. For example, as shown in Fig. 7, plaque distribution (displayed by distinguishing the affected area by color coding) and the degree of risk (representing the potential for disease using color density) can be display concurrently based on analysis results of plaque compositions from the blood vessel inner wall analyzing apparatus 1. In the case of displaying analysis results in real time in this manner, analyses can be carried out while confirming analysis results by referring to the monitor, for example, thereby making it possible to enhance the ease of use of the blood vessel inner wall analyzing apparatus 1 for a user.

In addition, the measurement light contained in the wavelength range of 1957 nm to 2713 nm used in the blood vessel inner wall analyzing apparatus 1, namely light in the near infrared wavelength range, has the characteristic of being resistant to scattering caused by erythrocytes and the like present in blood. Thus, the blood vessel inner wall analyzing apparatus 1 according to the present embodiment allows the obtaining of images of a measured position in a state in which blood flows through the blood vessel without interrupting blood flow as a result of expanding a blood vessel on the upstream side of the measured position with a balloon as in the case of conventionally used visible light vascular endoscopes, and without replacing the blood with physiological saline. Thus, according to the blood vessel inner wall analyzing apparatus 1, in addition to enabling analyses of measured positions to be carried out efficiently, the burden on the patient, attributable to treatment such as balloon expansion or physiological saline replacement, can be reduced.

Moreover, although a supercontinuum (SC) light source capable of outputting broadband light at high intensity without generating a large amount of heat is disclosed in Non-Patent Document 1 as an example of the light source 10 of the blood vessel inner wall analyzing apparatus 1, in the case of the use thereof, analysis of blood vessel inner walls can be carried out with higher precision.

Although the preceding has provided an explanation of the blood vessel inner wall analyzing apparatus 1 according to the present embodiment, the blood vessel inner wall analyzing apparatus according to the present invention is not limited to the above-mentioned embodiment, but rather can be altered in various ways. For example, although the case of the measurement target of the blood vessel inner wall analyzing apparatus being an inner wall of a carotid artery was explained with respect to the previously described embodiment, the blood vessel targeted for measurement is not limited to a carotid artery.

In addition, although the case of the light receiving fiber 40 being a fiber bundle was explained with respect to the previously described embodiment, the light receiving fiber 40 may also be a single optical fiber, for example. Moreover, although the case of the light illuminating fiber 30 and the light receiving fiber 40 inserted into the carotid artery C being comprised of mutually different optical fibers was explained with respect to the previously described embodiment, the functions of the light illuminating fiber 30 and the light receiving fiber 40 in the carotid artery C can also be realized using a single optical fiber. In this case, an optical fiber that directs light emitted from the spectrometer 20 to an optical fiber within the carotid artery C (measurement fiber) and an optical fiber that receives light from the measurement fiber and propagates reflected/scattered light that has propagated through the measurement fiber to the detector 50 are optically connected to one end of the measurement fiber. The other end of the measurement fiber serves as the end where measurement light is illuminated onto a measured position and reflected/scattered light enters from the measured position.

In addition, although a method for investigating plaque compositions was explained in the previously described embodiment that consists of measuring the spectrum within a specific wavelength range and carrying out second order differentiation on the result according to the wavelength to detect a peak originating in a specific component of plaque, it is not essential to determine the second derivative according to wavelength, but rather a mode can also be employed in which a composition is investigated based on the intensity of scattered/reflected light obtained by illuminating measurement light onto a measured position. In addition, a mode can also be employed in which, instead of measuring the scattering/reflection spectrum for near infrared light, the peak intensity of light that has passed through or been reflected by a measured position is determined by illuminating one specific or a plurality of specific wavelengths of near infrared light onto the measured position, and the ratio of components that constitutes plaque at the measured position are measured based on this result.

**Reference Signs List**

1: Blood vessel inner wall analyzing apparatus; 10: light source; 20: spectrometer; 30: light illuminating fiber; 40: light receiving fiber; 50: detection unit; and 60: analysis unit.

**Claims**

1. A blood vessel inner wall analyzing apparatus, comprising:

   illuminating means for illuminating at least a light component in a measurement wavelength range of 1957 nm to 2713 nm onto a measured position within a blood vessel, the illuminating means including a light emitting end to be inserted into the blood vessel and a light source that supplies the light component to be illuminated onto the measured position through the light emitting end;
   detecting means including a light entering end to be provided within the blood vessel and a detector that detects from the measured position the light component introduced through the light entering end; and
   analyzing means for analyzing compositions of the measured position based on intensity information on the light component detected by the detecting means,
   wherein the detecting means detects the light component in a detection wavelength range which extends 15 nm on both short and long sides of a selected center wavelength, with each of one or more center wavelengths being selected from a wavelength group comprised of 1972, 2055, 2057, 2141, 2167, 2172, 2182, 2212, 2257, 2259, 2270, 2274, 2282, 2289, 2294, 2309, 2311, 2324, 2334, 2345, 2348, 2360, 2371, 2375, 2385, 2388, 2402, 2416, 2443, 2468, 2469, 2488, 2504, 2510, 2544, 2565, 2607, 2628, 2630, 2656, 2668, 2678 and 2697 nm, and

the analyzing means analyzes for the presence or absence of substances differing from blood within the blood vessel by analyzing compositions of the measured position by using the intensity information on a light component in the detection wavelength range.

2. The blood vessel inner wall analyzing apparatus according to claim 1, wherein the light source of the illuminating means includes a supercontinuum light source.

3. The blood vessel inner wall analyzing apparatus according to claim 1 or 2, further comprising a spectrometer, arranged in the light path from the light source of the illuminating means to the detector of the detecting means, for separating at least the light component within the detection wavelength range out of the output light from the light source.

4. The blood vessel inner wall analyzing apparatus according to any one of claims 1 to 3, wherein the detecting means detects the light component in the detection wavelength range which extends 15 nm on both short and long sides of the selected center wavelength, with each of one or more center wavelength being selected from the wavelength group comprised of 1972, 2057, 2141, 2167, 2182, 2212, 2257, 2274, 2289, 2311, 2345, 2388, 2416, 2469, 2504, 2544 and 2630 nm, and

wherein the analyzing means analyzes for the presence or absence of thrombi and hematomas in a blood vessel by analyzing compositions of the measured position.

5. The blood vessel inner wall analyzing apparatus according to any one of claims 1 to 3, wherein the detecting means detects the light component in the detection wavelength range which extends 15 nm on both short and long sides of the selected center wavelength, with each of one or more center wavelengths being selected from the wavelength group comprised of 2055, 2172, 2259, 2270, 2282, 2294, 2309, 2324, 2334, 2348, 2360, 2371, 2375, 2385, 2402, 2443, 2468, 2488, 2510, 2565, 2607, 2628, 2656, 2668, 2678 and 2697 nm, and

wherein the analyzing means analyzes for the presence or absence of plaque in the blood vessel by analyzing compositions of the measured position.

6. The blood vessel inner wall analyzing apparatus according to any one of claims 1 to 5, wherein the detecting means detects, for three or more center wavelengths at least one of which is selected from said wavelength group, the light components belonging to the detection wavelength ranges which extend 15 nm on both short and long sides of the center wavelengths,

wherein the analyzing means calculates a spectral shape parameter to be used for identifying composition of the measured position, and analyzes the composition based on the results of the calculation, and

wherein the spectral shape parameter is either first derivative, normalized first derivative, second derivative or normalized second derivative of intensity information in each of the detection wavelength ranges and is calculated at the center wavelength.

7. The blood vessel inner wall analyzing apparatus according to claim 6, wherein the analyzing means calculates the second derivative at a central wavelength of intensity information in each of the detection wavelength ranges as a spectral shape parameter for identifying a composition of the measured position, and analyzes the composition based on a resulting calculation result.

8. The blood vessel inner wall analyzing apparatus according to claim 4, wherein the detecting means detects the light component contained in the detection wavelength range which extends 15 nm on both sides of the center wavelength of 1972 nm, and

wherein the analyzing means analyzes for the absence or presence of thrombi in the blood vessel by analyzing compositions of the measured position.

9. The blood vessel inner wall analyzing apparatus according to claim 5, wherein the detecting means detects, for one or more center wavelengths selected from the wavelength group comprised of 2309 nm and 2628 nm, the light components belonging to the detection wavelength ranges which extend 15 nm on both short and long sides of the center wavelengths, and

wherein the analyzing means analyzes for the presence or absence of an atheroma in the blood vessel by analyzing composition of the measured position.

10. The blood vessel inner wall analyzing apparatus according to claim 5, wherein the detecting means detects, for one or more center wavelengths selected from the wavelength group comprised of 2371 nm and 2402 nm, the light components belonging to the detection wavelength ranges which extend 15 nm on both short and long sides of the center wavelengths, and
wherein the analyzing means analyzes for the presence or absence of a lipid core in the blood vessel by analyzing composition of the measured position.

11. The blood vessel inner wall analyzing apparatus according to any one of claims 1 to 10, further comprising a display means for displaying an analysis result obtained from the analyzing means in real time.

12. Use of the blood vessel inner wall analyzing apparatus according to any one of claims 1 to 11, for analyzing compositions of a measured position within a blood vessel.

13. Use of a supercontinuum light source for the light source of the blood vessel inner wall analyzing apparatus according to any one of claims 1 to 11 that analyzes compositions of a measured position within a blood vessel.

14. A blood vessel inner wall analyzing method of analyzing compositions of a measured position within a blood vessel by using the blood vessel inner wall analyzing apparatus according to any one of claims 1 to 11, the method comprising the steps of:

inserting the light emitting end into the blood vessel so as to be positioned near the measured position;
installing the light entering end at a predetermined position within the blood vessel so that the light component from the measured position reaches the position;
illuminating at least the light component in the measurement wavelength range of 1957 nm to 2713 nm onto the measured position through the light emitting end;
detecting the light component that has entered through the light entering end from the measured position; and
generating analysis data generated for identifying the composition of the measured position from intensity information on the detected light component.

15. The blood vessel inner wall analyzing method according to claim 14, comprising the steps of:

detecting, for three or more center wavelengths at least one of which is selected from said wavelength group, the light components belonging to the detection wavelength ranges which extend 15 nm on both short and long sides of the center wavelengths, and
calculating a spectral shape parameter to be used for identifying composition of the measured position,
wherein the spectral shape parameter is either first derivative, normalized first derivative, second derivative or normalized second derivative of intensity information in each of the detection wavelength ranges and is calculated at the central wavelength.

16. The blood vessel inner wall analyzing method according to claim 14 or 15, wherein color-coded, two-dimensional tomographic images of the blood vessel are displayed in real time for each composition of a measured position.

*Fig.1*

EP 2 430 981 A1

*Fig.2*

*Fig.3*

# Fig.4

(A)

(B)

Fig.5

Fig.6

Fig.7

LIPID CORE
ATHEROMA
THROMBUS

## INTERNATIONAL SEARCH REPORT

| International application No. |
| --- |
| PCT/JP2010/058119 |

A. CLASSIFICATION OF SUBJECT MATTER
*A61B10/00*(2006.01)i, *G01N21/35*(2006.01)i

According to International Patent Classification (IPC) or to both national classification and IPC

B. FIELDS SEARCHED

Minimum documentation searched (classification system followed by classification symbols)
A61B10/00, G01N21/35

Documentation searched other than minimum documentation to the extent that such documents are included in the fields searched
Jitsuyo Shinan Koho  1922-1996 Jitsuyo Shinan Toroku Koho 1996-2010
Kokai Jitsuyo Shinan Koho 1971-2010 Toroku Jitsuyo Shinan Koho 1994-2010

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2007-531598 A (InfraReDx, Inc.),<br>08 November 2007 (08.11.2007),<br>paragraphs [0006], [0026], [0046] to [0054]<br>& US 2005/0228295 A1 & EP 1729634 A<br>& WO 2005/096921 A | 1,3-5,8-11<br>2 |
| X<br>Y | JP 2005-534415 A (InfraReDx, Inc.),<br>17 November 2005 (17.11.2005),<br>paragraphs [0020], [0040] to [0043], [0049],<br>[0052], [0053]<br>& US 2004/0024321 A1 & US 2004/0024298 A1<br>& US 2004/0077950 A1 & US 2008/0221455 A1<br>& EP 1538968 A  & EP 1551299 A<br>& WO 2004/012594 A1 & WO 2004/012586 A2<br>& WO 2004/012594 B & DE 60321470 D<br>& AT 397409 T | 1,3-5,8-11<br>2 |

☒ Further documents are listed in the continuation of Box C. ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br> 28 May, 2010 (28.05.10) | Date of mailing of the international search report<br> 08 June, 2010 (08.06.10) |
| --- | --- |
| Name and mailing address of the ISA/<br> Japanese Patent Office | Authorized officer |
| Facsimile No. | Telephone No. |

Form PCT/ISA/210 (second sheet) (July 2009)

| | INTERNATIONAL SEARCH REPORT | International application No. |
|---|---|---|
| | | PCT/JP2010/058119 |

C (Continuation).    DOCUMENTS CONSIDERED TO BE RELEVANT

| Category* | Citation of document, with indication, where appropriate, of the relevant passages | Relevant to claim No. |
|---|---|---|
| X<br>Y | JP 2007-503224 A  (InfraReDx, Inc.),<br>22 February 2007 (22.02.2007),<br>paragraphs [0003], [0023] to [0031], [0034]<br>& US 2005/0043637 A1     & EP 1658005 A<br>& WO 2005/018445 A1 | 1,3-5,8-11<br>2 |
| Y | JP 2008-229156 A  (Fuji Film Kabushiki Kaisha),<br>02 October 2008 (02.10.2008),<br>paragraph [0054]<br>(Family: none) | 2 |
| A | JP 2005-534428 A  (InfraReDx, Inc.),<br>17 November 2005 (17.11.2005),<br>entire text; all drawings<br>& US 2004/0024321 A1     & US 2004/0024298 A1<br>& US 2004/0077950 A1     & US 2008/0221455 A1<br>& EP 1551299 A          & EP 1538968 A<br>& WO 2004/012586 A2     & WO 2004/012594 A1<br>& WO 2004/012594 B      & DE 60321470 D<br>& AT 397409 T | 1-11 |

Form PCT/ISA/210 (continuation of second sheet) (July 2009)

| INTERNATIONAL SEARCH REPORT | International application No. |
|---|---|
| | PCT/JP2010/058119 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 12-16
   because they relate to subject matter not required to be searched by this Authority, namely:
   Claims 12 to 16 pertain to methods for treatment of the human body or animal body by surgery or therapy and diagnostic methods for the same, and thus relate to a subject matter on which the International Searching Authority
   (Continued to extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**          ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

| INTERNATIONAL SEARCH REPORT | International application No. |
|---|---|
| | PCT/JP2010/058119 |

Continuation of Box No.II-1 of continuation of first sheet(2)

is not required to carry out a search under the provisions of PCT Article 17(2)(a)(i) and PCT Rule 39.1(iv).

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004329550 A

- JP 2007185242 A

**Non-patent literature cited in the description**

- **Chenan Xia et al.** Mid-infrared supercontinuum generation to 4.5 $\mu$m in ZBLAN fluoride fibers by nanosecond diode pumping. *Optics Letters,* 01 September 2006, vol. 31 (17), 2553-2555